# EUROPÄISCHE PATENTSCHRIFT

(11) **EP 1 565 236 B1**
(45) Veröffentlichungstag und Bekanntmachung des Hinweises auf die Patenterteilung: **04.10.2006**
(21) Anmeldenummer: 03779960.8
(22) Anmeldetag: 17.11.2003
(51) Int. Cl.: A61N 1/39

(54) **DEFIBRILLATOR MIT VERBESSERTER ENDSTUFE**
DEFIBRILLATOR WITH IMPROVED OUTPUT STAGE
DEFIBRILLATEUR A ETAGE TERMINAL AMELIORE

(30) Priorität: 19.11.2002 DE 10254481
(43) Veröffentlichungstag der Anmeldung: 24.08.2005
(73) Patentinhaber: Metrax GmbH, D-78628 Rottweil (DE)
(72) Erfinder: BUCHER, Heinz, 78615 Rottweil (DE)
(74) Vertreter: Fleck, Hermann-Joseph
(86) Internationale Anmeldenummer: PCT/EP2003/012857
(87) Internationale Veröffentlichungsnummer: WO 2004/045714

(56) Entgegenhaltungen:
- EP-A- 0 569 616
- DE-A- 10 065 104
- US-A- 5 725 560
- US-A1- 2001 031 991
- US-A1- 2002 035 382
- US-B1- 6 208 896

## Beschreibung

Die Erfindung bezieht sich auf einen Defibrillator mit einer über eine Ansteuerschaltung zum Abgeben eines biphasischen Defibrillationspulses angesteuerten, eine H-Brücke zwischen einem Pluspol und einem Minuspol einer Energiespeichereinrichtung aufweisenden Endstufe, wobei ein Patientenstromkreis in dem mindestens eine Induktivität aufweisenden Querzweig ausgebildet ist und die biphasische Ansteuerung in an sich bekannter Weise durch wechselndes Schalten von in den vier H-Schenkeln der H-Brücke angeordneten Schaltgliedern zum Umkehren der Richtung des Patientenstromes im Querzweig erfolgt und wobei der Patientenstrom während der verschiedenen Phasen unter Vorgabe eines Sollwertes und Einbeziehung eines erfassten Istwertes mittels der Ansteuerschaltung durch Ansteuern der Schaltglieder-Anordnung mit einer höheren Frequenz als zur Erzeugung der beiden entgegengesetzten Phasen geregelt ist.

Ein Defibrillator dieser Art ist in der DE 100 65 104 A1 angegeben. Bei diesem bekannten Defibrillator mit einer gesteuerten Endstufe zum impulsartigen, biphasischen Beaufschlagen von an einen Patienten anzulegenden Elektroden mit elektrischer Energie aus einem Energiespeicher ist bei einer Ausführungsform eine H-Brücke vorgesehen, in deren Querzweig in Reihe ein Stromsensor, eine Induktivität in Form einer Spule sowie die Patientenelektroden mit dem Patientenwiderstand angeordnet sind. In den vier H-Schenkeln ist jeweils ein von einer Steuereinrichtung angesteuerter Halbleiterschalter angeordnet, über die mittels entsprechender Ansteuerung der Patientenstrom im Querzweig in zwei entgegengesetzten Richtungen steuerbar ist, wie in dieser Anmeldung näher beschrieben und an sich auch bekannt. Auf diese Weise werden für eine Defibrillation Impulse mit aufeinander folgenden entgegengesetzten Stromrichtungen erzeugt, die nach entsprechenden Erkenntnissen für das Herzgewebe verträglicher sind als monophasische Impulse gleicher Energie. Während der einzelnen Stromphasen wird der Strom durch Vergleich eines Istwertes des Patientenstromes mit einem Sollwert geregelt, wobei die Schalteranordnung mit einer höheren Frequenz als die Frequenz des biphasischen Pulses in näher angegebener Weise angesteuert wird. Bei den hohen Pulsspannungen von z.B. 1 kV oder höher und den hohen Frequenzen von z.B. mehr als 10 kHz für die Stromregelung bei einer Frequenz der biphasischen Impulse von z.B. einigen 100 Hz ist es schwierig, insbesondere die mit den höheren Frequenzen angesteuerten Schaltglieder geeignet zu steuern, so dass Fehler und Ausfälle der Endstufe nicht auszuschließen sind.

Der Erfindung liegt die Aufgabe zugrunde, einen Defibrillator der eingangs genannten Art so auszubilden, dass eine hohe Zuverlässigkeit bei der Abgabe biphasischer Defibrillationsimpulse erreicht wird.

Diese Aufgabe wird mit den Merkmalen des Anspruches 1 gelöst. Hiernach ist vorgesehen, dass zum Regeln des Patientenstromes in der einen Richtung nur das dieser Stromrichtung zugehörige Schaltglied in dem zum Minuspol zeigenden H-Schenkel mit der höheren Frequenz angesteuert ist, während zum Regeln des Patientenstromes in der anderen Richtung nur das dieser anderen Stromrichtung zugehörige Schaltglied in dem zum Pluspol zeigenden H-Schenkel mit der höheren Frequenz angesteuert ist, und dass antiparallel zu den mit höherer Frequenz angesteuerten Schaltgliedern jeweils mindestens eine Diode angeordnet ist, so dass über diese und das in der jeweiligen Phase dauernd geschlossene Schaltglied der Patientenstrom in seiner jeweiligen Richtung auch dann aufrecht erhalten wird, wenn das mit der höheren Frequenz angesteuerte Schaltglied sich in dem geöffneten Zustand befindet.

Mit diesen Maßnahmen wird zur Stromregelung in den beiden Phasen mit den entgegengesetzten Stromrichtungen beim Öffnen der mit höherer Frequenz angesteuerten Schaltglieder stets sichergestellt, dass der Patientenstrom in der während der betreffenden Phase gegebenen Richtung im Querzweig aufrecht erhalten wird, wobei der Stromkreis über die mindestens eine zu dem dauernd geöffneten Schaltglied antiparallele Diode und das während der betreffenden Phase dauernd geschlossene Schaltglied nach Art eines Freilaufkreises aufrecht erhalten wird. Die Stromregelung kann praktisch nach beliebigen Vorgaben über das mit der höheren Frequenz angesteuerte Schaltglied erfolgen und z.B. auch einer sich ändernden Sollwertvorgabe nachgeführt werden. Auch bei den hohen Ansteuerfrequenzen von z.B. einigen 10 oder 100 kHz und bei den hohen erforderlichen Spannungen von z.B. in der Größenordnung 1 kV oder höher ist ein sicherer Betrieb der Schaltglieder und damit der Endstufe sowie des gesamten Defibrillators gewährleistet.

Ein sicherer Betrieb bei einfachem Aufbau wird dadurch begünstigt, dass in dem Querzweig ein Stromsensor-Widerstand zum Erfassen des Patientenstromes angeordnet ist, dass aus dem Patientenstrom eine proportionale Spannung gebildet ist, die mittels eines Verstärkers verstärkt und als istwert einem Vergleich mit einer inneren Referenzspannung und einer äußeren Referenzspannung zugeführt ist, und dass bei Überschreiten der äußeren Referenzspannung ein Ansteuersignal der höheren Frequenz zum Öffnen des betreffenden Schaltglieds und bei Unterschreiten der inneren Referenzspannung ein Ansteuersignal der höheren Frequenz zum Schließen des betreffenden Schaltglieds gebildet ist. Die Einhaltung eines Sollstromes wird mittels Vorgabe der inneren und der äußeren Referenzspannung erreicht und entsprechend variierbar.

Dabei ergibt sich eine einfache, zuverlässige Ausbildung dadurch, dass das Ansteuersignal der höheren Frequenz über eine Logikschaltung gebildet ist. Für die Logikschaltung kommt z.B. ein programmierbarer Mikrocontroller oder ein Schaltnetz oder Schaltwerk mit Speichergliedern und Logikbausteinen in Betracht.

Zur Genauigkeit der Regelung tragen die Maßnahmen bei, dass die verstärkte proportionale Spannung vor, bei oder nach der Verstärkung gleichgerichtet ist.

Eine sichere Funktion der Endstufe wird dadurch unterstützt, dass an einem Anschlusspunkt im Querzweig zwischen einem Patientenwiderstand und der in Reihe dazu liegenden Induktivität einerseits zum Pluspol hin und andererseits zum Minuspol hin jeweils eine weitere Diodenanordnung bezüglich der Energiespeichereinrichtung jeweils in Sperrrichtung angeordnet ist. Hierdurch werden hohe transiente Spannungsimpulse in einer nachgeschalteten Koppelschaltung mit beispielsweise einem Koppel-Relais unterdrückt. Diese weiteren Diodenanordnungen bilden also keinen Teil der eigentlichen H-Brücke.

Sind zusätzlich auch die beiden Schaltglieder in den beiden übrigen H-Schenkeln mit antiparallel angeordneten Dioden überbrückt, werden negative Spitzen in den die Stromrichtung in den beiden verschiedenen Phasen bestimmenden übrigen Schaltgliedern unterdrückt.

Die Erfindung wird nachfolgend anhand eines Ausführungsbeispiels unter Bezugnahme auf die Zeichnungen näher erläutert. Es zeigen:
- Fig. 1: eine Endstufe und eine daran angeschlossene Ansteuerschaltung eines Defibrillators in schematischer Darstellung,
- Fig. 2: eine schematische Darstellung zur Funktion der Endstufe in einem Betriebszustand und
- Fig. 3: eine schematische Darstellung der Endstufe in einem anderen Betriebszustand.

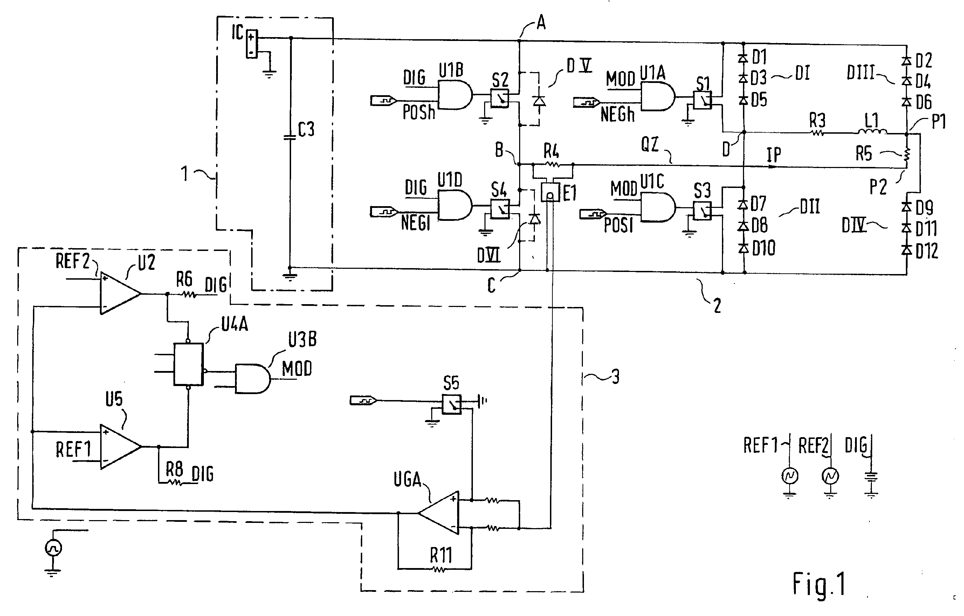

Fig. 1 zeigt als hier wesentliche Komponenten insbesondere eines tragbaren extern anwendbaren Defibrillators, eine Endstufe mit einer Energiespeichereinrichtung 1 und einem eien H-Brücke 2 aufweisenden Hochspannungsteil sowie eine an die Endstufe angeschlossene Ansteuerschaltung 3.

Die Energiespeichereinrichtung 1 kann in üblicher Weise, wie beispielsweise in der eingangs genannten DE 100 65 104 A1 näher angegeben, eine Ladeeinrichtung IC und eine an diese angeschlossene Energiespeichereinheit C3, wie etwa eine Kondensatoranordnung mit mindestens einem Kondensator oder einen Akkumulator, aufweisen. Die an die Energiespeichereinrichtung 1 angeschlossene H-Brücke 2 ist zur Erzeugung von biphasischen Defibrillationsimpulsen beispielsweise mit einer Frequenz von einigen 100 Hz ausgebildet, wobei die Spannung der Impulse z.B. in der Größenordnung von 1 kV oder höher liegen kann, wie an sich beispielsweise ebenfalls aus der genannten DE 100 65 104 A1 bekannt. Die biphasischen Impulse werden dabei dadurch gebildet, dass durch Ansteuern von Schaltgliedern, insbesondere Halbleiter-Schaltgliedern S1, S2, S3, S4, wie z.B. IGBTs, die in den H-Schenkeln angeordnet sind, in dem Querzweig QZ während der beiden Phasen des Defibrillationsimpulses ein Patientenstrom IP in zwei entgegen gesetzten Richtungen erzeugt wird. In dem Querzweig QZ sind die an den Patienten anzuschließenden Patientenelektroden oder ein entsprechender, wahlweise verbindbarer Patientenwiderstand R5 (z.B. zum Testen) in Reihe zu einem eine Induktivität L1 bildenden Schaltungsteil, insbesondere einer Spule oder einem äquivalenten Bauteil angeordnet. Außerdem liegt in dem Querzweig QZ ebenfalls in Reihe zu dem Patientenwiderstand R5 bzw. dem angeschlossenen Patienten ein Sensorwiderstand R4 zum Abgreifen eines Wertes für den Patientenstrom. Auch ein anderes Sensorelement ist denkbar. Vorliegend ist in dem Querzweig QZ noch ein weiterer in Reihe geschalteter Widerstand R3 vorgesehen.

Die zum Pluspol der Energiespeichereinrichtung 1 gerichteten H-Schenkel erstrecken sich somit zwischen einem Schaltungspunkt A auf der Seite des Pluspols und Schaltungspunkten B und D auf der Seite des Querzweigs QZ, während die zum Minuspol der Energiespeichereinrichtung 1 zeigenden H-Schenkel sich von den Schaltungspunkten B und D zu dem Schaltungspunkt C des Minuspols (Masse) der Energiespeichereinrichtung 1 erstrecken. Der Patientenwiderstand R5 bzw. stattdessen die Patientenelektroden liegen zwischen Anschlusspunkten P1, P2 des Querzweigs QZ. Die Schaltglieder S1, S2, S3, S4 werden über jeweilige Ansteuerglieder U1A, U1B, U1C bzw. U1D mittels Ansteuersignalen der Ansteuerschaltung 3 angesteuert. Zwischen den Schaltungspunkten A und D, und zwar antiparallel zu dem Schaltglied S1 einerseits und zwischen den Schaltungspunkten D und C, und zwar antiparallel zu dem Schaltglied S3 andererseits liegt jeweils eine Diodenanordnung DI und DII, die vorliegend aus mehreren Dioden D1, D3, D5 bzw. D7, D8, D10 zusammengesetzt sind. Durch die Antiparallelschaltung sperren die Diodenanordnungen DI und DII also den Stromfluss von dem Pluspol zu dem Minuspol der Energiespeichereinrichtung 1, so dass bei geöffneten Schaltgliedern S1, S2, S3, S4 kein Strom fließen kann. Zusätzliche Diodenanordnungen DIII und DIV in entsprechender Antiparallelschaltung sind zwischen dem Anschlusspunkt P1 und dem Schaltungspunkt A einerseits sowie dem Schaltungspunkt C andererseits angeordnet, wobei diese Diodenanordnungen DIII und DIV vorliegend ebenfalls aus mehreren einzelnen Dioden D2, D4, D6 bzw. D9, D11, D12 bestehen.

Dem Ansteuerglied U1B des Schaltgliedes S2 zwischen den Schaltungspunkten A, B sind Steuersignaleingänge für Signale DIG und POSh zugeordnet, während dem Ansteuerglied U1D Steuersignaleingänge für Steuersignale DIG und NEGI zugeordnet sind. Dem Ansteuerglied U1A des zwischen den Ansteuerpunkten A und D angeordneten Schaltglieds S1 sind Steuersignaleingänge für Steuersignale MOD, NEGh zugeordnet, während dem Ansteuerglied U1C für das Schaltglied S3 zwischen den Schaltungspunkten D, C Steuersignaleingänge für Steuersignale MOD und POSI zugeordnet sind. Auf diese Weise können die vier Schaltglieder einerseits zum Bilden der biphasischen Impulse und andererseits zur Stromregelung während der jeweiligen Phasen entgegengesetzter Stromrichtung in dem Querzweig QZ geeignet angesteuert werden, wobei die Steuersignale DIG und MOD von einer Stromregeleinrichtung der Ansteuerschaltung 3 gebildet und zugeführt werden.

Die Stromregeleinrichtung der Ansteuerschaltung 3 weist einen Stromrückkopplungszweig auf, der über dem Sensorwiderstand R4 eine proportionale Spannung E1 abgreift und einem Verstärker mit einem Schaltungselement U6A zuführt. Die dem Schaltungselement U6A bzw. dem damit gebildeten Verstärker zugeführte Spannung wird mittels eines Umschalters S5 gleichgerichtet, so dass die Spannung am Ausgang des Verstärkers mit dem Schaltungselement U6A in beiden Phasen gleiche Polarität besitzt und in gleicher Weise an Vergleichereinheiten U2, U5 angelegt und mit an deren Eingängen anliegenden Vorgabewerten REF2 bzw. REF1 verglichen werden kann. Dabei stellen die Vorgabewerte eine innere Referenzspannung REF1 bzw. eine äußere Referenzspannung REF2 dar, mit denen ein Regelintervall für den Sollstrom innerhalb eines Hysteresebandes gebildet wird. Ein Soll-Ist-Wert-Vergleich erfolgt somit in beiden Phasen in entsprechender Weise über die Vergleichereinheiten U2, U5. Die Vergleichsergebnisse werden einem Speicherglied U4A, beispielsweise einem bistabilen Speicherglied (Kippglied) zugeführt, an dessen einem Ausgang ein mit einem weiteren Steuereingang versehenes Logikglied U3B angeschlossen ist. Die Ausgänge der Vergleichereinheiten U2, U5 stellen über Widerstände R6 bzw. R8 das Steuersignal DIG zur Verfügung, während am Ausgang des Logikgliedes U3B das Steuersignal MOD vorliegt.

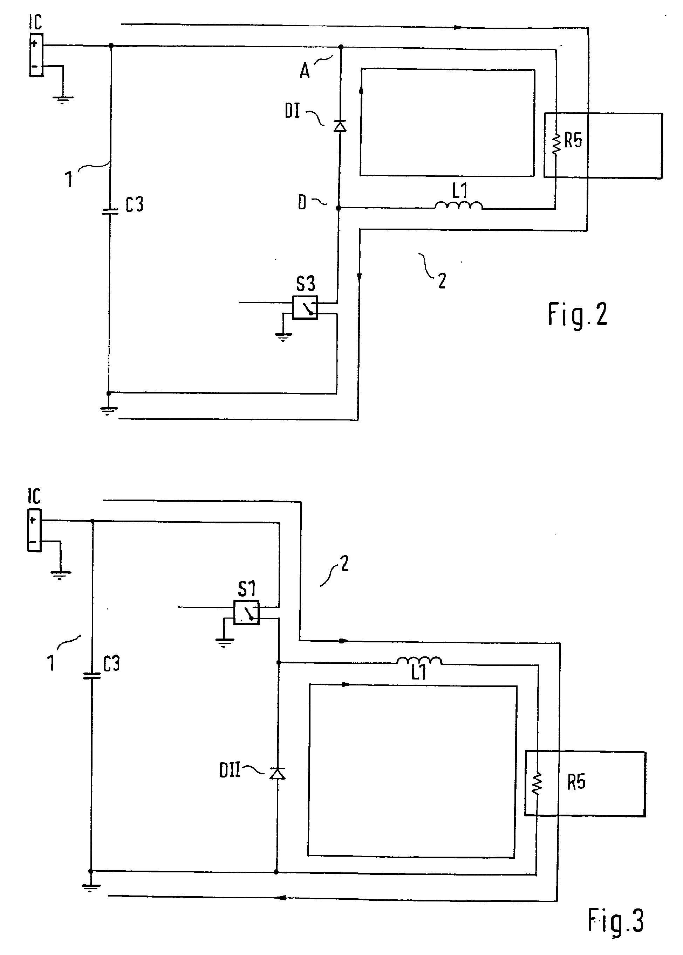

Für die Stromregelung in der einen Phase ist das Schaltglied S2 zwischen den Schaltungspunkten A und B dauernd geschlossen, während das Schaltglied S3 zwischen den Schaltungspunkten D und C mit einer höheren Frequenz des Steuersignals zum Regeln des Stroms angesteuert wird. Die beiden anderen Schaltglieder S1 zwischen den Schaltpunkten A und D und S4 zwischen den Schaltpunkten B und C sind in dieser Phase dauernd offen. Diese Situation ist in Fig. 2 dargestellt, wobei lediglich das in dieser Phase mit der höheren Frequenz angesteuerte Schaltglied S3 gezeigt ist, da das Schaltglied S2 dauernd geschlossen ist. Ist das Schaltglied S3 geschlossen, fließt der Patientenstrom durch den Patienten bzw. den Patientenwiderstand R5 vom Pluspol zum Minuspol der Energiespeichereinrichtung 1. Ist das Schaltglied S3 offen, fließt der Patientenstrom durch den Querzweig QZ in gleicher Richtung, jedoch durch die erste Diodenanordnung DI in dem H-Schenkel von dem Schaltungspunkt D nach A, wodurch ein Freilaufkreis gebildet ist. Die Ansteuerung des Schaltglieds S3 erfolgt dabei entsprechend der Stromregelung, wobei im geschlossenen Zustand des Schaltgliedes S3 der Strom von dem Energiespeicher C3 durch den Widerstand R5 bzw. die Impedanz des Patienten, die Induktivität L1 und das Schaltglied S3 ansteigt, bis der mittels der äußeren Referenzspannung REF2 gebildete Grenzwert erreicht ist. Wenn dann das Schaltglied S3 geöffnet wird, sinkt der Patientenstrom durch den Patientenwiderstand R5 bzw. den Patienten und die Induktivität L1 sowie die Diodenanordnung DI, bis der durch die innere Referenzspannung REF1 gebildete Referenzwert erreicht ist, wonach das Schaltglied S3 wieder geschlossen wird. Diese Regelungsvorgänge werden solange wiederholt, bis die betreffende Phase des Patientenstroms IP beendet ist, die z.B. einige oder einige zehn Millisekunden dauert.

Zum Bewirken der Phase mit entgegengesetztem Patientenstrom IP in dem Querzweig QZ werden die Schaltglieder S2, S3 geöffnet, während das Schaltglied S4 zwischen den Schaltungspunkten B und C dauernd geschlossen ist und das Schaltglied S1 zwischen den Schaltungspunkten D und A mit höherer Frequenz zum Regeln des Stroms angesteuert wird. Diese Situation ist in Fig. 3 dargestellt, wobei lediglich das hier relevante Schaltglied S1 eingezeichnet ist. Wenn das Schaltglied S1 im eingeschalteten Zustand ist, fließt der Patientenstrom IP von dem Energiespeicher C3 durch das Schaltglied S1, die Induktivität L1 und den Patientenwiderstand R5 bzw. den Patienten nach Masse. Wenn in diesem Zustand das Schaltglied S1 geöffnet wird, fließt der Patientenstrom IP in entsprechender Richtung weiter durch den Patientenwiderstand R5 bzw. den Patienten, die Induktivität L1 sowie die Diodenanordnung DII, wodurch ebenfalls ein Freilaufkreis gebildet wird. Dieser Zustand hält solange an, bis der der inneren Referenzspannung REF1 entsprechende Wert des Patientenstroms IP erreicht wird, wonach mittels der Stromregelschaltung das Schaltglied S1 wieder geschlossen wird und so fort, bis diese entgegengesetzte Phase des Patientenstroms IP bzw. des Defibrillationsimpulses beendet wird. Die verschiedenen Impulsphasen können abwechselnd mit einer geeigneten Anzahl wiederholt werden.

Durch Vorgabe entsprechender Referenzwerte REF1, REF2 kann die Stromregelung in gewünschter Weise beeinflusst werden (Bestimmung der Breite und Form des Hysteresebandes), wobei mit der beschriebenen Ausbildung der H-Brücke und deren Ansteuerung ein zuverlässiger Betrieb des Defibrillators erreicht wird.

Auch die in den entsprechenden Phasen dauernd eingeschalteten Schaltglieder S2 bzw. S4 in den H-Schenkein A-B bzw. B-C können mit Dioden DV bzw. DVI in Antiparallelanordnung überbrückt werden, wodurch negative Spannungsüberhöhungen in diesen Schaltgliedern S2 bzw. S4 unterdrückt werden.

## Patentansprüche

1. Defibrillator mit einer über eine Ansteuerschaltung (3) zum Abgeben eines biphasischen Defibrillationspulses angesteuerten, eine H-Brücke (2) zwischen einem Pluspol und einem Minuspol einer Energiespeichereinrichtung (1) aufweisenden Endstufe, wobei ein Patientenstromkreis in dem mindestens eine Induktivität (L1) aufweisenden Querzweig (QZ) ausgebildet ist und die biphasische Ansteuerung durch wechselndes Schalten von in den vier H-Schenkeln der H-Brücke (2) angeordneten Schaltgliedern (S1, S2, S3, S4) zum Umkehren der Richtung des Patientenstromes (IP) im Querzweig (QZ) erfolgt und wobei der Patientenstrom (IP) während der verschiedenen Phasen unter Vorgabe eines Sollwertes und Einbeziehung eines erfassten Istwertes mittels der Ansteuerschaltung (3) durch Ansteuern der Schaltglieder-Anordnung (S1, S2, S3, S4) mit einer höheren Frequenz als zur Erzeugung der beiden entgegengesetzten Phasen geregelt ist,
**dadurch gekennzeichnet,**
**dass** zum Regeln des Patientenstromes (IP) in der einen Richtung nur das dieser Stromrichtung zugehörige Schaltglied (S3) in dem zum Minuspol zeigenden H-Schenkel (D-C) mit der höheren Frequenz angesteuert ist, während zum Regeln des Patientenstromes (IP) in der anderen Richtung nur das dieser anderen Stromrichtung zugehörige Schaltglied (S1) in dem zum Pluspol zeigenden H-Schenkel (D-A) mit der höheren Frequenz angesteuert ist, und
**dass** antiparallel zu den mit höherer Frequenz angesteuerten Schaltgliedern (S3, S1) jeweils mindestens eine Diode (DII, DI) angeordnet ist, so dass über diese und das in der jeweiligen Phase dauernd geschlossene Schaltglied (S2, S4) der Patientenstrom (IP) in seiner jeweiligen Richtung auch dann aufrecht erhalten wird, wenn das mit der höheren Frequenz angesteuerte Schaltglied (S3, S1) sich in dem geöffneten Zustand befindet.

2. Defibrillator nach Anspruch 1,
**dadurch gekennzeichnet,**
**dass** in dem Querzweig (QZ) ein Stromsensor-Widerstand (R4) zum Erfassen des Patientenstromes (IP) angeordnet ist,
**dass** aus dem Patientenstrom (IP) eine proportionale Spannung (E1) gebildet ist, die mittels eines Verstärkers (U6A) verstärkt und als Istwert einem Vergleich mit einer inneren Referenzspannung (REF1) und einer äußeren Referenzspannung (REF2) zugeführt ist, und
**dass** bei Überschreiten der äußeren Referenzspannung (REF2) ein Ansteuersignal der höheren Frequenz zum Öffnen des betreffenden Schaltglieds (S3, S1) und bei Unterschreiten der inneren Referenzspannung (REF1) ein Ansteuersignal der höheren Frequenz zum Schließen des betreffenden Schaltglieds (S3, S1) gebildet ist.

3. Defibrillator nach Anspruch 2,
**dadurch gekennzeichnet,**
**dass** das Ansteuersignal der höheren Frequenz über eine Logikschaltung (U4A, U3B) gebildet ist.

4. Defibrillator nach Anspruch 2 oder 3,
**dadurch gekennzeichnet,**
**dass** die verstärkte proportionale Spannung (E1) vor, bei oder nach der Verstärkung gleichgerichtet ist.

5. Defibrillator nach einem der vorhergehenden Ansprüche,
**dadurch gekennzeichnet,**
**dass** an einem Anschlusspunkt (P1) im Querzweig (QZ) zwischen einem Patientenwiderstand (R5) und der in Reihe dazu liegenden Induktivität (L1) einerseits zum Pluspol hin und andererseits zum Minuspol hin jeweils eine weitere Diodenanordnung (DIII, DIV) bezüglich der Energiespeichereinrichtung jeweils in Sperrrichtung angeordnet ist.

6. Defibrillator nach einem der vorhergehenden Ansprüche,
**dadurch gekennzeichnet,**
**dass** auch die beiden Schaltglieder (S2, S4) in den beiden übrigen H-Schenkeln (A-B, B-C) mit antiparallel angeordneten Dioden (DV, DVI) überbrückt sind.

## Claims

1. Defibrillator having an output stage which is triggered via a trigger circuit (3) to emit a biphased defibrillation pulse and has an H-bridge (2) between a positive pole and a negative pole of an energy storage unit (1), a patient circuit being formed in the transverse branch (QZ) having at least one inductive resistor (L1) and the biphased triggering taking place by varied switching of switching members (S1, S2, S3, S4) arranged in the four H-legs of the H-bridge (2) for reversing the direction of the patient current (IP) in the transverse branch (QZ), and the patient current (IP) being controlled during the various phases by presetting a desired value and incorporating a detected actual value by means of the trigger circuit (3) by triggering the switching member arrangement (S1, S2, S3, S4) with a higher frequency than that for generating the two opposite phases,
**characterised in that**
for regulating the patient current (IP) in the one direction, only the switching member (S3) assigned to this current direction in the H-leg (D-C) pointing to the negative pole is triggered at the higher frequency, while for regulating the patient current (IP) in the other direction only the switching member (S1) assigned to this other current direction in the H-leg (D-A) pointing to the positive pole is triggered at the higher frequency, and
**in that** anti-parallel with the switching members (S3, S1) triggered at the higher frequency, in each case at least one diode (DII, DI) is arranged, such that, via this diode and the switching member (S2,S4) continuously closed in the respective phase, the patient current (IP) is maintained in its respective direction even if the switching member (S3, S1) triggered at the higher frequency is in an open state.

2. Defibrillator according to claim 1, **characterised**
**in that** a current sensor resistor (R4) for detecting the patient current (IP) is arranged in the transverse branch (QZ),
**in that** from the patient current (IP) is formed a proportional voltage (E1) which is amplified by means of an amplifier (U6A) and supplied as an actual value for a comparison with an internal reference voltage (REF1) and an external reference voltage (REF2), and
**in that**, if the external reference voltage (REF2) is exceeded, a trigger signal of the higher frequency is formed for opening the respective switching member (S3,S1), and, if the internal reference voltage (REF1) is not reached, a trigger signal of the higher frequency is formed for closing the respective switching member (S3, S1).

3. Defibrillator according to claim 2, **characterised**
**in that** the trigger signal of the higher frequency is formed via a logic circuit (U4A, U3B).

4. Defibrillator according to claim 2 or 3,
**characterised in that** the amplified proportional voltage (E1) is rectified before, during or after the amplification.

5. Defibrillator according to one of the preceding claims, **characterised in that** at a connecting point (P1) in the transverse branch (QZ) between a patient resistor (R5) and the inductive resistor (L1) placed in series therewith, a further diode arrangement (DIII, DIV) is respectively arranged in the blocking direction with regard to the energy storage device on the one hand toward the positive pole and on the other hand toward the negative pole.

6. Defibrillator according to one of the preceding claims, **characterised in that** the two switching members (S2, S4) in the two remaining H-legs (A-B, B-C) are also bridged by diodes (DV, DVI) arranged anti-parallel.

## Revendications

1. Défibrillateur avec un étage terminal qui est activé par l'intermédiaire d'un circuit d'activation (3) dans le but de générer une impulsion biphasée de défibrillation et qui comprend un pont en H (2) entre un pôle plus et un pôle moins d'un dispositif d'accumulation d'énergie (1), dans lequel un circuit de courant utilisé pour le patient est formé dans la branche transversale (QZ) comprenant au moins une inductance (L1) et l'activation biphasée pour inverser la direction du courant appliqué au patient (IP) a lieu dans la branche transversale (QZ) par une commutation alternative d'éléments de commutation (S1, S2, S3, S4) disposés dans les quatre branches en H du pont en H (2) et dans lequel le courant appliqué au patient (IP) est, une valeur de consigne étant imposée et une valeur réelle détectée étant intégrée, régulé lors des différentes phases au moyen du circuit d'activation (3) par l'activation de l'ensemble d'éléments de commutation (S1, S2, S3, S4) avec une fréquence plus élevée que pour la génération des deux phases opposées,
**caractérisé**
**en ce que**, pour la régulation dans l'une des directions du courant appliqué au patient (IP), seul l'élément de commutation (S3) qui est associé à cette direction du courant est activé avec la fréquence plus élevée dans la branche en H (D-C) dirigée vers le pôle moins tandis que, pour la régulation dans l'autre direction du courant appliqué au patient (IP), seul l'élément de commutation (S1) qui est associé à cette autre direction du courant est activé avec la fréquence plus élevée dans la branche en H (D-A) dirigée vers le pôle plus et
**en ce que** dans le sens antiparallèle par rapport aux éléments de commutation (S3, S1) activés avec une fréquence plus élevée est disposée respectivement au moins une diode (DII, DI), de sorte que, par l'intermédiaire de celle-ci et de l'élément de commutation (S2, S4) qui est fermé en permanence lors de la phase respective, le courant appliqué au patient (IP) est aussi maintenu dans sa direction respective lorsque l'élément de commutation (S3, S1) activé avec la fréquence plus élevée se trouve dans la position d'ouverture.

2. Défibrillateur selon la revendication 1,
**caractérisé**
**en ce qu'**une résistance de capteur de courant (R4) servant à capter le courant appliqué au patient (IP) est disposée dans la branche transversale (QZ),
**en ce qu'**à partir du courant appliqué au patient (IP) est formée une tension proportionnelle (E1), laquelle est amplifiée au moyen d'un amplificateur (U6A) et est amenée en tant que valeur réelle à un élément de comparaison avec une tension interne de référence (REF1) et avec une tension externe de référence (REF2) et
**en ce qu'**en cas de dépassement de la tension externe de référence (REF2) est formé un signal d'activation de la fréquence plus élevée pour l'ouverture de l'élément de commutation correspondant (S3, S1) et qu'en cas de non atteinte de la tension interne de référence (REF1) est formé un signal d'activation de la fréquence plus élevée pour la fermeture de l'élément de commutation correspondant (S3, S1).

3. Défibrillateur selon la revendication 2,
**caractérisé**
**en ce que** le signal d'activation de la fréquence plus élevée est formé par l'intermédiaire d'un circuit logique (U4A, U3B).

4. Défibrillateur selon la revendication 2 ou 3,
**caractérisé**
**en ce que** la tension proportionnelle amplifiée (E1) est redressée avant, pendant ou après l'amplification.

5. Défibrillateur selon l'une quelconque des revendications précédentes,
**caractérisé**
**en ce qu'**à un point de raccordement (P1) dans la branche transversale (QZ), entre une résistance concernant le patient (R5) et l'inductance (L1) placée en série avec celle-ci, est disposé respectivement dans le sens du blocage, d'une part en direction du pôle plus et d'autre part en direction du pôle moins, respectivement un autre agencement de diodes (DIII, DIV) se rapportant au dispositif d'accumulation d'énergie.

6. Défibrillateur selon l'une quelconque des revendications précédentes,
**caractérisé**
**en ce que** les deux éléments de commutation (S2, S4) sont également shuntés dans les deux autres branches en H (A-B, B-C) au moyen de diodes (DV, DVI) disposées dans le sens antiparallèle.
